Europäisches Patentamt

European Patent Office

Office européen des brevets

(19)

(11) Publication number: **0 154 815 B1**

## EUROPEAN PATENT SPECIFICATION

(12)

(45) Date of publication of patent specification: **13.03.91**  (51) Int. Cl.⁵: **A61B 17/115**

(21) Application number: **85101426.6**

(22) Date of filing: **11.02.85**

(54) **Improved skin stapler.**

(30) Priority: **12.03.84 US 588415**
**12.03.84 US 588275**

(43) Date of publication of application:
**18.09.85 Bulletin 85/38**

(45) Publication of the grant of the patent:
**13.03.91 Bulletin 91/11**

(84) Designated Contracting States:
**BE CH DE FR GB IT LI NL SE**

(56) References cited:
**EP-A- 0 094 752**
**US-A- 2 744 251**
**US-A- 4 180 196**
**US-A- 4 470 532**

(73) Proprietor: **AMERICAN CYANAMID COMPANY**
**1937 West Main Street P.O. Box 60**
**Stamford Connecticut 06904-0060(US)**

(72) Inventor: **Lehmann, K. Li**
**305 Barlow Road**
**Fairfield Connecticut 06430(US)**
Inventor: **Marra, Michael c/o American**
**Cyanamid Company**
**1937 West Main Street**
**Stamford, Conn. 06904-0060(US)**

(74) Representative: **Wächtershäuser, Günter, Dr.**
**Tal 29**
**W-8000 München 2(DE)**

## Description

This invention relates to a surgical skin/fascia stapling instrument according to the preamble of Claim 1.

Such an instrument is known from US-A-4 180 156. The instrument contains a plurality of staples and allows a single staple to be formed and removed from the instrument.

According to the present invention, an improved surgical stapler has been invented having the features defined in the characterizing clause of Claim 1.

In the inventive embodiment, the distal portion of the first track is curved toward the second track such that the staple moves on the first track to the anvil flange.

In a preferred embodiment of the improved stapler the anvil flange is movable and is in substantial alignment with at least the first track before the staple moves to the flange. The anvil surface is substantially parallel to the tracks.

## DESCRIPTION OF THE DRAWINGS

Figure 1 is a partially cutaway side view showing the improvements, and a means to control the movement of the trigger into the handle;

Figure 2 is an expanded side view of the lower portion of the magazine circled in Figure 1;

Figure 3 is an expanded, partially cutaway and perspective view of the circled portion shown in Figure 1, and showing the improvements;

Figures 4 to 6 are expanded, partially cutaway and perspective views showing the improvements;

Figure 7 is an expanded, partially cutaway and perspective view showing the relationship of the improvements.

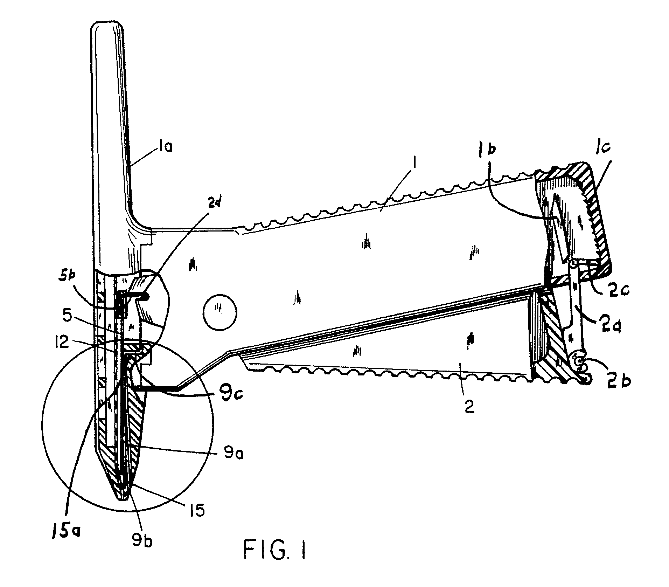

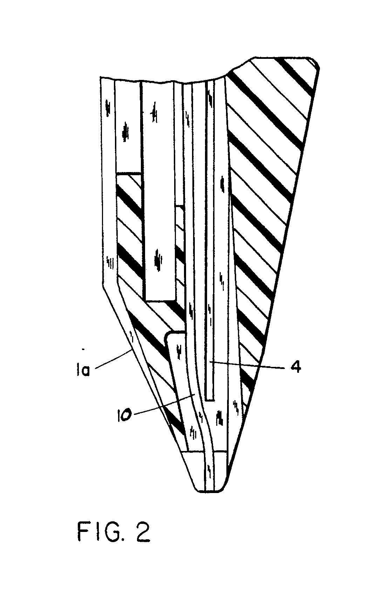

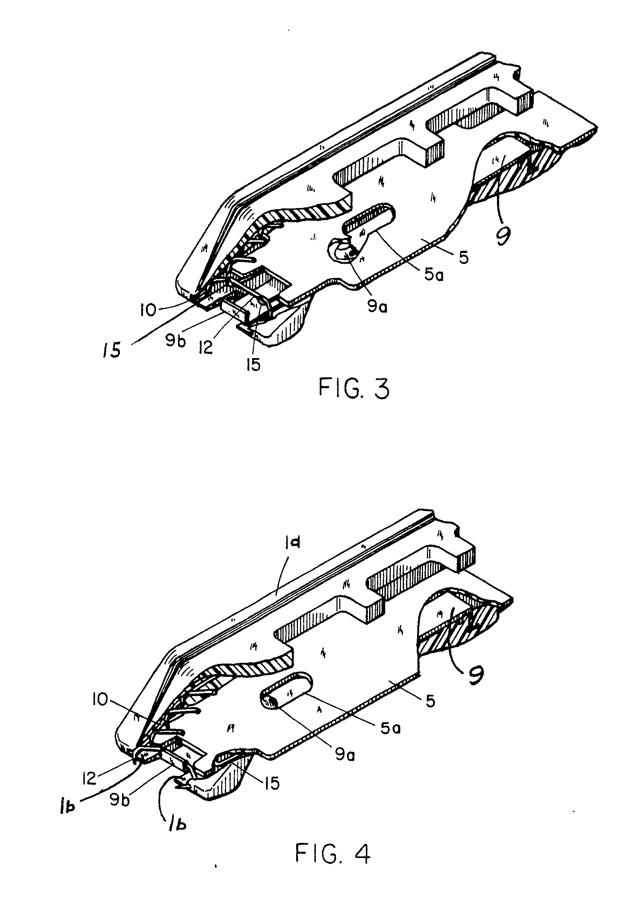

Referring to Figure 1, trigger 2 is fully extended from handle 1. In Figures 1 and 3 (Figure 3 is an expanded, partially cutaway and perspective view of the circled portion shown in Figure 1), the instrument is in its rest or static condition. In Figures 1 and 2, the loading of the staples 12 into the staple track 10 is by feeding one staple behind the other in a point to crown configuration.

Referring again to Figure 1, the orientation of the magazine 1a (and therefore the anvil shelf 9b in the magazine) to the handle 1 and/or the trigger 2 is not critical to the practice of this invention. That is, the orientation can be essentially perpendicular, (as shown in Figure 1), parallel or oblique. Also, the orientation of the anvil shelf 9b to the handle 1 and/or the trigger 2 can be variable, e.g. by pivotally attaching the magazine 1a to the handle 1 and/or trigger 2.

The proximal ends of the anvil surface 9c and the hold back spring 15a can be separately or jointly imbedded in the magazine 1a by techniques known in the prior art, e.g. by molding one or more grooves in the magazine 1a.

Referring to Figure 3, forming blade 5 is at its fully retracted position. With the forming blade 5 in this position, raised cam 9a on anvil surface 9' is out of the hole 5a of forming blade 5. The anvil shelf 9b is out of alignment with staple 12 and forming blade 5. It is to be understood that the terms "cam" and "boss", as used to describe element 9a, are synonomous. It is further to be understood that the terms "hole" and "opening", as used to describe element 5a, are synonomous. Also the terms "anvil flange" and "anvil shelf" as used to describe element 9b are synonymous.

Referring further to Figure 3, hold back spring 15 is in its undeflected position. There are two structurally and functionally identical elements 15 shown in figure 3 for the hold back spring. However, the elements 15 can be made as one piece. Also, the hold back spring can be made as one structural element. Therefore, the two elements 15 are jointly described as a hold back spring. The first staple 12 in the column of staples rests on hold back spring 15.

Until the forming blade 5 moves the first staple 12, the hold back spring 15 remains in its undeflected position and holds the column of staples in the track 10. In this position the hold back spring 15 offsets a negatory spring force on the column of staples. This negatory spring force is a permanent bias to the column of staples.

The use of a negatory spring as a bias for a plurality of staples is known in the prior art. The use of a sinusoidal advancing spring for a column of staples is also known from the prior art.

Referring to Figure 1, the forming blade 5 can be retracted by a flange 5b on the proximal end of the forming blade. The flange fits into a groove 2d in the trigger. The motion of the forming blade 5 is thus directly dependent on the motion of the trigger 2.

In Figures 1, 2 and 4, as trigger 2 is compressed into handle 1, the forming blade 5 is caused to advance in track 4 (shown in Figure 2) until the beading edge contacts the top of the first staple 12 in the track 10. With advance of the forming blade 5, the anvil surface cam 9a falls into hole 5a in the forming blade. This causes the anvil surface 9 to relax from its flexed position. This relaxation brings the anvil forming shelf 9b into alignment with the staple 12 in the staple track 10 and with the forming blade 5 in the forming track 4.

Preferably, the cam 9a falls into hole 5a before or as the distal portion of forming blade 5 contacts the first staple 12. This functional relationship is preferred because it allows for the anvil shelf 9b to be in alignment with the forming blade 5 and with the first staple 12 before the first staple is stripped off the hold back spring 15. That is, the anvil shelf 9b is in position to receive the first staple 12 before the first staple is stripped off the hold back spring 15.

The position of the anvil surface 9 and shelf 9b is dependent on the position of the cam 9a to the opening 5a. That is, the linear motion of the forming blade 5 causes the opening 5a to act on the cam 9a. This action simultaneously causes arcuate motion of the anvil surface 9 and the shelf 9b.

After the cam 9a moves into the opening 5a, the structural relationship between the forming blade 5 and the anvil shelf 9b is fixed, and they are in substantial alignment in the plane of the forming blade's linear motion.

Referring generally to Figures 1 and 4 to 7, the improved stapler can have a means to control the movement of the trigger 2 into the handle 1. An adequate control means is disclosed in the prior art and is shown in Figure 1 in this application. Referring to Figure 1, a trigger ratchet pawl 2c indirectly contacts at least one cam 1b in handle 1. This pushes the ratchet pawl 2c into a ratchet 1c. With the pawl 2c held against the ratchet 1c, trigger 2 is held in its position even though the trigger squeezing force is released.

Further, arm 2a supporting pawl 2c can be movably attached to trigger 2, e.g. as shown in Figure 1 pivot 2b. Alternatively, arm 2a can be permanently attached to trigger 2. Finally, as shown in Figure 1 in this application, the scalloping on the contacting edges of the handle 1 and trigger 2 is optional. It is to be understood that these contacting edges can be of any geometrical configuration, i.e. straight or contoured, which will allow the trigger 2 to be compressible into the handle 1.

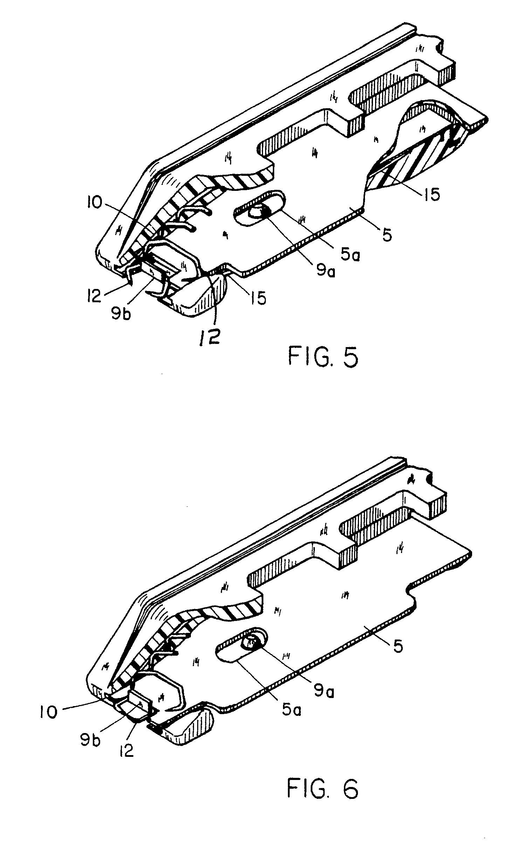

Referring to Figures 4 and 5, further compressing of the trigger 2 into the handle 1 causes the forming blade 5 to further advance. In Figure 4, the staple deflects hold back spring 15, allowing escapement of the first staple 12 from the column of staples. The hold back spring 15 is held in its deflected position by being in contact with a surface of the forming blade 5. In Figure 5, forming blade 5 carries the first staple 12 on track 10 forward until it is positioned against the anvil shelf 9b. The trigger pawl 2c, as shown in Figure 1 and as disclosed in the above prior art references, is engaged by a ratchet 1c during the motion shown in Figure 4.

Referring specifically to Figure 4, the staple track 10 continues to hold the legs of the first staple 12 even though the points of the staple protrude from the tip of the magazine 1a. In the preferred embodiment, staple track 10 sequentially carries the first staple 12 from the column of staples to the anvil shelf 9b. However, another embodiment can be that the first staple 12 in the column of staples enters forming track 4 (shown in Figure 2) and is then carried on track 4 to the anvil shelf 9b.

The height of the anvil shelf 9b is equal to or greater than the cross-sectional width of the staple 12. In many embodiments, because the staples in the column of staples will be circular in cross-section, the width of the staple will be equal to its diameter. If the height of the shelf is equal to the cross-sectional width or diameter of the first staple, the travel of the anvil shelf 9b from the stripping surface 1b (on the magazine 1a) to a position substantially in alignment with the first staple 12 and the forming blade 5 is essentially equal to the height of the anvil shelf 9b. If the height of the anvil shelf is greater than the cross-sectional width or diameter of the staple, the travel of the anvil shelf 9b is essentially equal to or less than the height of the anvil shelf.

As shown in Figure 5, a surface of the forming blade 5 retains the column of staples 12 in the staple track 10. In summary, and as also shown in Figures 2, 4 and 6, one side of the forming blade 5 holds the column of staples 12, and the other side of the forming blade holds spring 15 from moving, after the forming blade 5 is advanced past the hold back spring 15.

In Figure, the trigger 2 is squeezed into handle 1 to the end of its stroke. This causes the forming blade 5 to fully advance, which bends the first staple 12, around anvil shelf 9b until it is fully formed. The forming of a staple around an anvil flange is well known in the prior art.

Anvil shelf 9b remains in alignment with the forming blade 5. The two legs of the formed staple perpendicular to the crown are backed by a staple stripping area 1b (more fully shown in Figure 4) at the distal end of magazine 1a.

The trigger ratchet pawl 2c becomes inactive as or after the forming blade 5 is fully advanced. This is because the ratchet pawl indirectly falls off the cam 1b, allowing the trigger 2 to return to its initial rest position.

In Figure 6, hold back spring 15 and the column of staples 12 continue to be held by the forming blade 5.

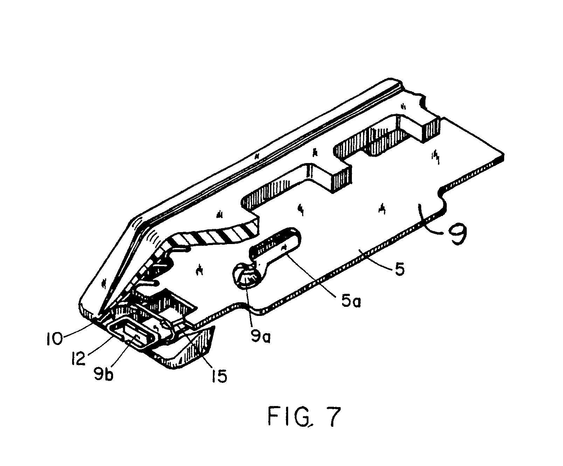

In Figures 1 and 7, the trigger 2 is released. This allows the trigger 2 to return to its initial (fully opened) position. As stated above in describing Figure 6, this is because the trigger pawl 2c is functionally inoperative after the pawl rides over the cam 1b.

Referring specifically to Figure 7, the forming blade 5 retracts. This allows the hold back spring 15 to relax and move into its initial rest position. In this position and after the forming blade 5 retracts a sufficient distance, the second staple in the column of staples moves down the staple track 10 until it is arrested by the hold back spring 15. It is to be understood that the remaining staples in the column of staples simultaneously move down the track with the second staple.

At approximately the end of the forming blade stroke, the anvil cam 9a is forced to ride out of the hole 5a. This causes the anvil surface 9 and the shelf 9b to flex toward the position shown in Figure 3. As the anvil shelf 9b flexes, the formed staple 12 is separated from the anvil shelf 9b by the stripping surface 1b (more fully shown in Figure 4) on the magazine 1a.

Referring to Figures 6 and 7, the bottom of the stripping surface 1b is in a plane equal to or below one-half of the cross-sectional height of the staple crown 12 (on the anvil shelf 9b). For a staple having a circular cross-section, one-half the height is equal to the radius.

## Claims

1. A surgical stapler having an anvil surface (9) terminating in an anvil flange (9b), a first track (10) movably containing a plurality of staples and a second track (4) movably containing a forming blade (5), said first track being substantially parallel to said second track, the distal portion of said first track and the distal portion of said second track being so configured that a staple (12) from said plurality of staples can move to said anvil flange (9b) and said forming blade (5) can form said staple (12) around said flange, characterized in that the distal portion of said first track (10) is curved toward said second track (4) to be in substantial alignment with the distal portion of said second track (4) such that said staple (12) moves on said first track (10) to said anvil flange (9b).

2. The surgical stapler of Claim 1, wherein said anvil flange (9b) is movable and is in substantial alignment with at least said first track (10) before said staple (12) moves to said flange (9b).

## Revendications

1. Agrafeuse chirurgicale, comprenant une surface de semelle (5), se terminant par une patte de semelle (9b), une première glissière (10), contenant de manière mobile un grand nombre d'agrafes, et une seconde glissière contenant de manière mobile une lame de mise en forme (5), la première glissière étant pratiquement parallèle à la seconde glissière et la partie distale de la première glissière et la partie distale de la seconde glissière étant conformées de façon qu'une agrafe (12) provenant d'un groupe d'agrafes puisse se déplacer jusqu'à la patte de semelle (9b) et que la lame de mise en forme (5) puisse mettre cette agrafe (12) en forme autour de cette patte, caractérisée en ce que la partie distale de la première glissière (10) est incurvée en direction de la seconde glissière (4) de façon à être pratiquement dans l'alignement de la partie distale de cette seconde glissière (4) de manière que l'agrafe (12) se déplace sur la première glissière (10) jusqu'à la patte de semelle (9b).

2. Agrafeuse chirurgicale suivant la revendication 1, dans laquelle la patte de semelle (9b) est mobile et est pratiquement alignée avec au moins la première glissière (10) avant que l'agrafe (12) ne se déplace jusqu'à cette patte (9b).

## Ansprüche

1. Chirurgisches Klammerinstrument mit einer in einem Amboßflansch (9b) endenden Amboßoberfläche (9), einer ersten Schiene (10), in der eine Vielzahl von Klammern bewegbar gehalten wird und eine zweite Schiene (4), in der ein Formgebungsorgan (5) bewegbar gehalten wird, wobei die erste Schiene im wesentlichen parallel zu der zweiten Schiene verläuft, der distale Abschnitt der ersten Schiene und der distale Abschnitt der zweiten Schiene so konfiguriert sind, daß eine Klammer (12) von der erwähnten Vielzahl von Klammern sich zu dem Amboßflansch (9b) bewegen kann und das Formgebungsorgan (5) die Klammer (12) um den Flansch herum formen kann, dadurch gekennzeichnet, daß der distale Abschnitt der ersten Schiene (10) zur zweiten Schiene (4) hin gekrümmt ist, so daß er mit dem distalen Abschnitt der zweiten Schiene (4) im wesentlichen fluchtet, derart, daß sich die Klammer (12) auf der ersten Schiene (10) zu dem Amboßflansch (9b) bewegt.

2. Chirurgisches Klammerinstrument gemäß Anspruch 1, wobei der Amboßflansch (9b) bewegbar ist und zumindest mit der ersten Schiene (10) im wesentlichen fluchtet, bevor sich die Klammer (12) zu dem Flansch (9b) bewegt.

FIG. 1

FIG. 2

FIG. 3

FIG. 4

7

FIG. 5

FIG. 6

8

FIG. 7